# EUROPEAN PATENT SPECIFICATION

(11) **EP 1 713 468 B1**
(45) Date of publication and mention of the grant of the patent: **05.05.2010**
(21) Application number: 05706144.2
(22) Date of filing: 01.02.2005
(51) Int. Cl.: A61K 31/365, A61K 31/4184, A61P 33/10, A61K 47/36, A61K 47/02

(54) **ANTHELMINTIC COMPOSITION**
WURMMITTEL
COMPOSITION ANTHELMINTHIQUE

(30) Priority: 03.02.2004 US 541472 P
(43) Date of publication of application: 25.10.2006
(73) Proprietor: Wyeth LLC, Madison, NJ 07940 (US)
(72) Inventor: ROWE, James, S., Rockdale, NSW 2216 (US); HAYES, Jon, C., East Brunswick, NJ 08816 (US)
(74) Representative: Wileman, David Francis
(86) International application number: PCT/US2005/002794
(87) International publication number: WO 2005/074914

(56) References cited:
- EP-A- 0 717 993
- WO-A-00/61068
- WO-A-00/74489
- WO-A-03/092680
- WO-A-2004/043445
- GB-A- 2 252 730

## Description

### TECHNICAL FIELD

The invention relates to anthelmintic compositions combining two or more active compounds and which are particularly adapted for use in veterinary applications.

### BACKGROUND OF THE INVENTION

Any discussion of the prior art throughout the specification should in no way be considered as an admission that such prior art is widely known or forms part of common general knowledge in the field.

Helminthiasis is a widely occurring disease affecting animals, particularly warm-blooded animals, causing substantial economic losses: Particularly susceptible to the infections are sheep, cattle, goats, horses, and other domesticated herbivores. Many know anthelmintic agents have been discovered possessing varying degrees of efficacy on the particular helminths causing the infections. Certain classes of anthelmintics have a greater or lesser spectrum of activity, *i.e*., they are able to treat infections involving a wider or smaller range of parasistes.

A particularly useful class of anthelmintics are those of the avermectin and milbemycin classes, exemplified by abamectin, ivermectin, doramectin, eprinomectin, milbemycin D and moxidectin. These have activity against parasitic roundworms and also against some ectoparasites, but lack activity against cestodes (tapeworms), and against trematodes (flukes). U.K Patent Specifications 2166436, 2176182 and 2187742 and EP 170006 describe antibiotic compounds, designated Antibiotics S541, prepared by fermentation of Streptomyces microorganisms and/or chemical derivatives thereof.

Such compounds have antibiotic, and, in particular, anti-endoparasitic, anti-ectoparasitic, anti-fungal, insecticidal, nematicidal and acaricidal activity and are of special use in agriculture, horticulture and animal and human health. These compounds include the avermectin and milbemycin groups of compounds, and are frequently referred to as endectocides.
Liver fluke is a global disease which mainly infects cattle and sheep but can also develop in many other animals including horses, pigs, goats, rabbits and at least in Australia, native animals such as kangaroos and wombats. Humans may also be infected with liver fluke. Liver fluke can cause serious economic losses. Global losses due to liver fluke disease are estimated at over three billion US dollars per year. In sheep, infection with liver fluke reduces production, including wool growth and wool quality, lambing percentages and growth rates of lamb. Sheep can also die as a result of liver fluke infection.

Triclabendazole is another useful anthelmintic, described in U. S. Patent 4,197,307, having activity primarily *vis* a *vis* trematodes, and particularly against liver flukes. It can attack early immature fluke, immature fluke and adult liver fluke. It is normally supplied as a separate oral drench due to its formulation requirements. However, it is time consuming and costly to provide separate drenches for the treatment of each category of disease-producing parasites.

A combination of actives which would have the combined spectrum of activity of the avermectin/milbemycins and triclabendazole is obviously desirable. However, successful combination formulations must provide for physical stability of the formulation for a commercially reasonable period of time; chemical stability of the actives therein; maintain or exceed the level of pharmacological activity of the individual actives, and be administrable to the animal in a suitable dosage form.

Liquid formulations of therapeutic agents may be in the form of a solution or a suspension. Solutions are liquid preparations that contain one or more chemical substances dissolved in a suitable solvent or mixture of mutually miscible solvents. Substances in solutions are more susceptible to chemical instability than those in the solid state. However, solutions are sometimes required for bioavailability. Drugs that are essentially insoluble in pharmaceutically acceptable liquids, and particularly water, are conveniently formulated as suspensions. Merriam-Webster's Dictionary gives the meaning of "insoluble" as "incapable of being dissolved in a liquid; also: soluble only with difficulty or to a slight degree". Thus, pharmaceutical suspensions are liquid preparations that consist of solid particles dispersed throughout a liquid phase in which the particles are not soluble. The compositions of the present invention are generally liquid formulations or formulations adapted to be constituted as a liquid formulation by addition of an appropriate vehicle, usually, and most preferably, water.

By its very nature, the particular matter in a suspension may settle or sediment to the bottom of the container upon standing. Such sedimentation may also lead to caking and solidification of the sediment with a resulting difficulty in redispersing the suspension upon agitation. To prevent such problems, suitable ingredients that increase viscosity and the gel state of the suspension, such as clays, surfactants, polyols, polymers, or sugars, can be incorporated in the formulation. Nonetheless, it is necessary to consider that the suspension must have the necessary stability, as well as fluidity, across ambient temperature ranges.

When formulating anthelmintic compositions it is necessary that the compositions maintain the chemical stability of the active compounds, as well as the physical stability of the formulation. This allows for the compositions to be prepared well in advance of their intended uses and to have a useful shelf life as a commercial product.

EP 329460 discloses that the stability of avermectin/milbermycins can be enhanced in the presence of an antioxidant. In particular, EP 329460 refers to the stabilisation of a group of Antibiotic S541 derivatives described in UK 2192630A and in particular to 23[E]-methoxyimino Factor A, also known as moxidectin. A variety of antioxidants are disclosed in EP 329460 as useful for stabilisation with particular reference to butylated hydroxytoluene (BHT). These antioxidants are disclosed as being present in amounts ranging from 0.005 to 1 % with respect to the antibiotic compounds.

Australian Patent No. 78276/01 discloses a stable anthelmintic composition comprising an avermectin or milbemycin formulated with praziquantel. The composition is stabilised using about 0.15% to about 5% of a stabilising antioxidant such as butylated hydroxytoluene. This composition is stable, however, the active praziquantel does not provide a treatment of liver fluke.

It is an object of the present invention to overcome or ameliorate the disadvantages of the prior art, or to provide a useful alternative.

### DISCLOSURE OF THE INVENTION

In a broad aspect, the present invention provides an anthelmintic oral drench composition comprising a milbemycin in combination with triclabendazole, and a rheology modifier.

In another aspect, the present invention provides a method of rendering an anthelmintic composition suitable for use as an oral drench comprising the addition of a non-surfactant rheology modifier.

In a further preferred embodiment, the amount of the non-surfactant rheology modifier utilized in the composition is sufficient to render the viscosity in the range of 1650 to 2050 Mpa, with variance from this as ±20%, and to maintain appropriate viscosity characteristics over the temperature ranges of from about 4 °C to about 40 °C.

The present invention thus provides a mechanism of combining a milbemycin, *e.g*., moxidectin, with triclabendazole in order to provide a veterinary composition which treats and controls both roundworms (nematodes) and liver fluke (trematodes) in a single dosage form. By combining the known therapeutic range of a milbemycin, *e.g*., moxidectin, with triclabendazole, a formulation is provided with dual efficacy, in the convenient dosage form of an oral drench, and possessing the necessary chemical and physical stability in storage and use.

In a further preferred embodiment, the non-surfactant rheology modifier is an inorganic rheology modifier, or a high molecular weight polysaccharide rheology modifier. It preferably contains one or more of the following components: silicon dioxide, magnesium oxide, alginates, cellulose or other polysaccharides, or mixtures thereof.

A highly preferred rheology modifier is a composition containing sodium silicate, and particularly the synthetic sodium silicate compositions commercially available as Laponite, for instance, Laponite® RDS. (Laponite® is a Registered Trade Mark of Southern Clay Products Inc, Gonzales, Texas, United States of America). Laponite® is described as a layered sodium silicate composition which is processed with sodium, magnesium and lithium to produce an amorphous precipitate that is then crystallised by a high temperature treatment. It is available in several distinct presentations, with the sol forming types such as those of the grades DS, being most suitable for use in the present invention. Laponite ® RDS hydrates and swells in water to give a clear and colourless colloidal dispersion of low viscosity. It is taught to be particularly useful as a rheology modifier for household and industrial cleaners, but surprisingly, it has been found to be suitable for use to modify the physical properties/viscosity of anthelmintic compositions, while maintaining the physical and chemical stability of both types of actives for periods that represent commercial shelf lives for veterinary products. It also is suitably non-toxic to be able to be utilized without safety concerns in food-producing animals.

A preferred Laponite ® is Laponite ® RDS hydrous sodium lithium magnesium silicate modified with tetra sodium pyrophosphate which has the following chemical analysis:

| | |
|---|---|
| SiO₂ | 54.5 |
| MgO | 26.0 |
| Li₂O | 0.8 |
| Na₂O | 5.6 |
| P₂O₅ | 4.1 |
| Loss on Ignition | 8.0 |

Typical physical properties are as follows:

| Appearance | Free flowing white powder |
|---|---|
| Bulk Density, kg/m³ | 1000 |
| Surface Area (BET), m²/g | 330 |
| pH (2% suspension) | 9.7 |
| Sieve Analysis, % < 250 microns | 98 |
| Moisture Content, % | 10.0 |

A further highly preferred non-surfactant rheology modifier is xanthan gum, a high molecular weight polysaccharide produced by the bacterium *Xanthomonas campestris* found on cabbage plants. Xanthan gum is particularly desirable due to its high stabilizing properties, high viscosity at low concentration, solublity in both hot and cold water, high pseudoplasticity, excellent freeze/thaw stability, and resistance to pH and temperature variations.

Depending upon the particular rheology modifier used, the amount used in the present invention may vary from about 0.1 to about 2% by weigh/volume, with amounts in the range of 0.2 to about 2% by weight/volume being especially preferred. For the preferred Laponite® RDS grade, amounts in the range of about 0.5-1.0%, and particularly 0.7% weight/volume are highly preferred. In order to utilize the Laponite in conjunction with the other ingredients of the formulation, it is preferred that it be added to the formulation as a solution in water of approximately 20% of the total water before coming into contact with the formulation.

Stability and viscosity are to some extent competing interests. It is necessary to provide both chemical and physical stability as well as suitable rheology for using the formulation as an oral drench. Simply adding triclabendazole to a known milbemycin formulation does not provide a formulation with the necessary stability and commercial usability.

The Applicants have determined that it is necessary to provide a quantity of the non-surfactant rheology modifier sufficient to maintain the combined moxidectin/triclabendazole formulation in a form that is suitable for use as an oral drench. Oral drenches are typically administered via a drenching gun, which requires that the formulation be neither too thin nor too viscous. Various rheology modifiers may be used, however, the Laponite® RDS or xanthan gum are most suitable as the rheology modifier since they not only provide suitable rheological effect, especially over the range of temperatures, but also provide for chemical stability of the active agents of the formulation whilst maintaining a physically stable composition. The formulation thus retains consistency in its administered dose of, each of the two actives, and appropriate consistency of the fluid so as to not clog or jam the drenching apparatus. Most importantly, since the actives themselves are not degraded over the shelf life of the product, the farmer or veterinarian can be assured that the labeled doses of active are administered to each individual animal.

As will be understood by persons skilled in the art, providing such a formulation with dual efficacy in a suitable dosage form that is also physically and chemically stable in storage and use is a complex problem. Making a high viscosity formulation may provide physical stability but may cause difficulties with dosage. Chemical stability of the formulation is also vital and accordingly, it is necessary that the rheology modifier has minimal effect on the chemical stability of the each of the actives, as well as the formulation itself.

In still a further aspect the present invention provides a method of treating a mammal comprising providing an efficacious dose of the aforementioned anthelmintic composition.

### BEST MODE(S) FOR CARRYING OUT THE INVENTION

The present invention relates to anthelmintic compositions and method of improving or modifying the effectiveness of such compositions in an oral drench form.

Typical endectocide compounds for use in the compositions according to the invention are the milbemycins (*e.g*., milbemycin D, moxidectin).

Highly preferred for use in the formulations of the present invention are moxidectin technical material and moxidextin technical concentrate, the former which includes the antioxidant BHT. This technical material or concentrate can be stored for a suitable length of time in order that commercial products can be prepared which include the active material. For example, the Fort Dodge Animal Health products CYDECTIN® and VETDECTIN® liquid compositions in New Zealand are solutions prepared using moxidectin technical material or moxidectin technical concentrate. Both these commercial products have excellent stability.

Both CYDECTIN® and VETDECTIN® include as active material, moxidectin, stabilised by the BHT originally present in the technical material or concentrate of moxidectin used to produce the commercial product.

It was surprisingly found that triclabendazole could not simply be added to an existing milbemycin formulation and used as an oral drench. While not wishing to be bound by any theory, it is believed that the typical milbemycin formulation having appropriate stability characteristics also has properties which interfere with the suspension of the triclabendazole active. The Applicants determined, however, when combined with a suitable non-surfactant rheological additive, a moxidectin/triclabendazole formulation could be provided which was chemically stable, physically stable and had a viscosity which allowed it to be used as an oral drench.

The formulations of the present invention provide a solution of the milbemycin in which the triclabendazole is suspended. Most advantageously, the chemical integrity of both actives is preserved, and the physical condition of the formulation has a commercially acceptable shelf life whereby the actives and other constituents remain in the liquid without undesirable settling out or breakdown of the formulation which would result in an unacceptable variability of dosing animals with the product. Typical administration of the compositions of the present invention is via the use of a drench gun. For such administration, the composition must necessarily have some viscosity, but not so much as to cause jamming of the gun or have complicated storage requirements.

Preferably, the compositions of the present invention comprise:
0.01-5% wt/vol (w/v) of milbemycin;
sufficient amounts of one or more organic solvents and one or more surfactants to enable the dissolution of the milbemycin;
1-10% wt./vol (w/v) triclabendazole;
0.1-2% wt./vol (w/v) of a rheology modifier;
40-80% (w/v) water; and optionally, solvents, surfactants, buffering agents, and preservatives.

As will be clear to persons skilled in the art, where compositions according to the present invention are to be used or be prepared for use, in veterinary medicine, they may also contain additional carriers, stabilizing agents, buffering agents, preservatives or other excipients as will be well known in the art.

The formulations of the present invention should be formulated to administer a dose of the milbemycin suitable for the treatment of roundworms and a dose of triclabendazole sufficient to treat or prevent liver flukes. For the milbemycins, the dose will range from about 0.01 to about 0.5 mg/kg of animal body weight, with a dose of 0.4 mg/kg body weight being highly preferred for moxidectin. In a preferred embodiment, this will mean a concentration of about 1.0 mg/mL moxidectin in the liquid formulation of the present invention.

The triclabendazole component of the composition of the present invention is typically present in an amount of about 1-10% by weight/volume of the composition, and is generally selected so as to provide the animal receiving the composition with a dosage of about 10-50 mg/kg of animal body weight. Typically, the triclabendazole is utilized as fine particles of 10-50 microns in size, with it being highly preferred that the triclabendazole is micronized so that 90% of the particles are less than 10 microns in size. This maximizes the absorption of the triclabendazole, and achieved a suitable blood level to enable the desired level of therapeutic activity.

The avermectin or milbemycin of the present formulation is solubilized in the water of the formulation by using one or more particular organic solvents and one or more surfactants as solubilizers. Generally, such solvents and surfactants are selected for their ability to solubilize the particular milbemycin in minimal amounts, as well as for their compatibility with the particular milbemycin. Preferred solvents for solubilizing moxidectin are, for instance, propylene glycol, glycerol formal, glycerine and polyethylene glycol, with propylene glycol and polyethylene glycol, especially polyethylene glycol 6000, being highly preferred. Preferred surfactants are the nonionic surface-active agents such as polyoxyethylated vegetable oils, polyoxyethylene sorbitan monoisostearate, polyoxyethylene sorbitan monostearate and polyoxyethyene sorbitan monooleate (also known as polysorbate 80, and sold under the trademark Tween® 80).

Optionally, but usually, buffering systems can be utilized to maintain the pH of the formulation at optimally between 6.0 and 6.8. Preferred are buffer combinations such as sodium phosphate dibasic dodecahydrate and sodium phosphate monobasic dihyrate. In the manufacturing process where very large commercial quantities are being prepared, the use of an antifoam agent such as Antifoam 9020 is also highly preferred.

The compositions of the present invention typically also contain suitable antimicrobial agents to protect against bacteria, yeast and mould contamination. As examples of antimicrobial preservatives there may be mentioned methyl-,ethyl-, propyl- and butyl-parabens, benzyl alcohol, sodium edetate, or combinations thereof. Typical and highly preferred stabilizing agents are those such as butylated hydroxytoluene (BHT).

In another embodiment, the present invention provides a modified formulation which includes selenium. Selenium is an important additive for the treatment of "white muscle disease," a degenerative condition of the heart. In some areas, food stocks are selenium deficient. The formulation may be modified with small quantities of selenium, *e.g*., up to around 0.1%. The selenium can be added in by any suitable method. One particularly suitable method is by the use of sodium selenate.

The present invention will now be described with reference to the following examples. These are the embodiments of the present invention and should in no way be considered limiting.

### EXAMPLES

The following examples illustrate the invention and include preferred forms of the invention. Moxidectin can be supplied via a moxidectin technical concentrate in benzyl alcohol (30%) [MTC], or, as moxidectin technical [90%] material (MTM).

Examples 1 and 2 are oral drench formulations using moxidectin technical concentrate and moxidectin technical material respectively. It will be noted that in both examples, 0.70 %wt/vol of Laponite® RDS is used and preferably, BHT (butylated hydroxy toluene) is added in a quantity of 0.25%w/v to assist in providing a stable formulation. Examples 3 and 4 are oral drench formulations using moxidectin technical concentrate and moxidectin technical material, respectively, with xanthan gum being utilized as the rheology modifier.

### EXAMPLE I

### MOXIDECTIN AND TRICLABENDAZOLE ORAL DRENCH

| Label Amount | | | Raw Materials | | | |
|---|---|---|---|---|---|---|
| Ingredient | Amount (%w/v) | % Excess | Component Description | Requirements | | |
| | | | | Potency | %w/v | kg/ 1000L |
| Moxidectin | 0.1% | 5.0 | Moxidectin Technical Concentrate | 30% | 0.345 | 3.45 |
| Triclabendazole | 5.0% | 5.0 | Triclabendazole | 100% | 5.250 | 52.5 |
| | | | Antifoam 9020 | | 0.070 | 0.70 |
| | | | Polysorbate 80* | | 10.000 | 100.00 |
| | | | Butylated Hydroxytoluene** | | 0.250 | 2.5 |
| | | | Disodium EDTA* | | 0.200 | 2.0 |
| | | | Propylene Glycol* | | 10.000 | 100.00 |
| | | | Polyethylene Glycol 6000** | | 3.000 | 30.00 |
| | | | Polyoxyethylene 40 stearate** | | 2.500 | 25.0 |
| | | | Sodium Phosphate Dibasic Dodecahydrate* | | 0.950 | 9.50 |
| | | | Sodium Phosphate Monobasic Dihydrate* | | 0.620 | 6.20 |
| | | | Benzyl Alcohol** | | 1.000 | 10.00 |
| | | | Laponite RDS ® | | 0.70 | 7.00 |
| | | | Purified Water USP | | q.s. | 701.1 |
| | | | | | | |
| | | | | Total | 105.0 | 1050.0 |

| | | | | | | |
|---|---|---|---|---|---|---|
| * USP or equivalent ** NF or equivalent | | | | | | |

The Laponite® RDS is combined with an amount of water equivalent to 20% w/v Laponite RDS, mixed until fully dispersed, and then heated to 70-80 °C. To this is then added the polyethylene glycol 6000, polyoxyethylene 40 stearate and butylated hydroxytoluene, with mixing until homogenous. The buffering agents are then added, with further mixing, and to the resultant mixture is added polysorbate 80, antifoam 9020, followed by a mixture of the triclabendazole and the propylene glycol. When mixing is complete, a solution of the moxidectin in benzyl alcohol is added, followed by the remainder of water to make volume. The pH is adjusted to the range 6.0-6.8 before filling into packaging.

### EXAMPLE II

### MOXIDECTIN AND TRICLABENDAZOLE ORAL DRENCH

| Label Amount | | | Raw Materials | | | |
|---|---|---|---|---|---|---|
| Ingredient | Amount (%w/v) | % Excess | Component Description | Requirements | | |
| | | | | Potency | %w/v | kg/ |
| | | | | | | 1000L |
| Moxidectin | 0.1% | 5.0 | Moxidectin Technical Material | 90% | 0.1167 | 1.167 |
| Triclabendazole | 5.0% | 5.0 | Triclabendazole | 100% | 5.250 | 52.5 |
| | | | Antifoam 9020 | | 0.070 | 0.70 |
| | | | Polysorbate 80* | | 10.000 | 100.00 |
| | | | Butylated Hydroxytoluene** | | 0.250 | 2.5 |
| | | | Disodium EDTA* | | 0.200 | 2.0 |
| | | | Propylene Glycol* | | 10.000 | 100.00 |
| | | | Polyethylene Glycol 6000** | | 3.000 | 30.00 |
| | | | Polyoxyethylene 40 stearate** | | 2.500 | 25.0 |
| | | | Sodium Phosphate Dibasic Dodecahydrate* | | 0.950 | 9.50 |
| | | | Sodium Phosphate Monobasic Dihydrate* | | 0.620 | 620 |
| | | | Benzyl Alcohol** | | 1.228 | 12.28 |
| | | | Laponite RDS ® | | 0.70 | 7.00 |
| | | | Purified Water USP | | q.s. | 701.1 |
| | | | | | | |
| | | | | Total | 105.0 | 1050.0 |

| | | | | | | |
|---|---|---|---|---|---|---|
| *USP or equivalent **NF or equivalent | | | | | | |

The above ingredients are mixed in the same order as those of Example I. The pH is adjusted, if outside the range of 6.0 - 6.8, with 10% Sodium Hydroxide solution or 20% Phosphoric acid solution before filling into final packaging.

### EXAMPLE III

### MOXIDECTIN AND TRICLABENDAZOLE ORAL DRENCH

| Label Amount | | | Raw Materials | | | |
|---|---|---|---|---|---|---|
| Ingredient | Amount (%w/v) | % Exces s | Component Description | Requirements | | |
| | | | | Potency | %w/v | kg/ |
| | | | | | | 1000L |
| Moxidectin | 0.1% | 5.0 | Moxidectin Technical Concentrate | 30% | 0.345 | 3.45 |
| Triclabendazole | 5.0% | 5.0 | Triclabendazole | 100% | 5.250 | 52.5 |
| | | | Antifoam 9020 | | 0.070 | 0.70 |
| | | | Polysorbate 80* | | 10.000 | 100.00 |
| | | | Butylated Hydroxytoluene** | | 0.250 | 2.5 |
| | | | Disodium EDTA* | | 0.200 | 2.0 |
| | | | Propylene Glycol* | | 10.000 | 100.00 |
| | | | Polyethylene Glycol 6000** | | 3.000 | 30.00 |
| | | | Polyoxyethylene 40 stearate** | | 2.500 | 25.0 |
| | | | Sodium Phosphate Dibasic Dodecahydrate* | | 0.950 | 9.50 |
| | | | Sodium Phosphate Monobasic Dihydrate* | | 0.620 | 6.20 |
| | | | Benzyl Alcohol** | | 1.000 | 10.00 |
| | | | Xanthan Gum** | | 0.250 | 2.50 |
| | | | Purified Water* | | q.s. | 705.65 |

| | | | | | | |
|---|---|---|---|---|---|---|
| *USP or equivalent **NF or equivalent | | | | | | |

The polyethylene glycol 6000, polyoxyethylene 40 stearate and butylated hydroxytoluene are added to approximately 70% of the water, which has been heated to about 70 °C, with mixing until homogenous. The buffering agents are then added, with further mixing, followed by addition of the polysorbate 80, and antifoam 9020.

In a separate vessel, the moxidectin Technical Concentrate is added to the benzyl alcohol with mixing, and then the Xanthan gum is added, with further mixing.

The triclabendazole is mixed with propylene glycol, and the resultant mixture is added to the water mixture, with stirring. After reducing the temperature of the water solution to about 40 °C, the moxidectin technical concentratelbenzyl alcohol mixture is added, followed by a further reduction of the temperature to about 25 °C, and addition of water to make volume. The pH is adjusted to the range 6.0-6.8 before filling into packaging with 10% Sodium Hydroxide solution or 20% Phosphoric acid solution.

### EXAMPLE IV

### MOXIDECTIN AND TRICLABENDAZOLE ORAL DRENCH

| Label Amount | | | Raw Materials | | | |
|---|---|---|---|---|---|---|
| Ingredient | Amount (%w/v) | % Excess | Component Description | Requirements | | |
| | | | | Potency | %w/v | kg/ |
| | | | | | | 1000L |
| Moxidectin | 0.1% | 5.0 | Moxidectin Technical Material | 90% | 0.1167 | 1.167 |
| Triclabendazole | 5.0% | 5.0 | Triclabendazole | 100% | 5.250 | 52.5 |
| | | | Antifoam 9020 | | 0.070 | 0.70 |
| | | | Polysorbate 80* | | 10.000 | 100.00 |
| | | | Butylated Hydroxytoluene** | | 0.250 | 2.5 |
| | | | Disodium EDTA* | | 0.200 | 2.0 |
| | | | Propylene Glycol* | | 10.000 | 100.00 |
| | | | Polyethylene Glycol 6000* | | 3.000 | 30.00 |
| | | | Polyoxyethylene 40 stearate** | | 2.500 | 25.0 |
| | | | Sodium Phosphate Dibasic Dodecahydrate * | | 0.950 | 9.50 |
| | | | Sodium Phosphate Monobasic Dihydrate * | | 0.620 | 6.20 |
| | | | Benzyl Alcohol ** | | 1.228 | 12.28 |
| | | | Xanthan Gum ** | | 0.250 | 2.50 |
| | | | Purified Water* | | q.s. | 705.65 |

| | | | | | | |
|---|---|---|---|---|---|---|
| *USP or equivalent ** NF or equivalent | | | | | | |

The above ingredients are mixed in the same order as those of Example I. The pH is adjusted, if outside the range of 6.0 - 6.8, with 10% Sodium Hydroxide solution or 20% Phosphoric acid solution before filling into final packaging.

### EXAMPLE V

### STABILITY STUDIES - MOXIDECTIN/TRICLABENDAZOLE ORAL DRENCH

The stability of the formulations described shown in Examples 1-4 were tested over an extensive period. Various batches were placed in packaging material 80% LDPE/20% HDPE. As this packaging material is known to be weaker than 100% HDPE, it is representative of the worst case scenario.

Additionally, the batches were stored at different temperatures, 25°C, 30°C, and 40°C to see what effect this would have on the stability of the product.

Moxidectin was assayed by HPLC. Triclabendazole was assayed by HPLC.
The appearance was determined by visual inspection and the pH of the sample was determined by direct potentiometric measurement of the sample. Specific gravity was measured directly using a Specific Gravity instrument.

### RESULTS

The label claim for moxidectin in Moxidectin and Triclabendazole oral drench is 0.1 % w/v and that for triclabendazole is 5% w/v. The moxidectin content, triclabendazole content, appearance, specific gravity at 25°C and pH at 25°C for the formulation are given below.

Over the test time period, ie over 24 months, the moxidectin concentration for various batches of formulation varied in the range 89.0 - 105.4% of label claim for the accelerated study at 40°C, (ambient humidity), 91.0 - 108.6% of label claim for the real time study at 30°C, (ambient humidity), and 101.0 -108.6% of label claim for the real time study at 25°C, ambient humidity. The acceptable range of results for moxidectin concentration is 95.0 - 110.0% of label claim. For batch No 1, the moxidectin content was within specification at both 25 and 30°C after 18 months. While for batch No 2, the formulation was V0418610 is within specification after 9 months at both 25 and 30°C. For batch No 3, it appeared it was out of specification after 9 months at 30°C. Later testing 12 months confirmed this trend.

The triclabendazole concentration for the three batches of formulation varied in the range 103.5 - 107.3% of label claim for the accelerated study at 40°C, (ambient humidity), 96.9 - 107.5% of label claim for the real time study at 30°C, (ambient humidity), and 99.0 -107.3% of label claim for the real time study at 25°C, (ambient humidity). The acceptable range of results for triclabendazole concentration is 90.0 - 110.0% of label claim. The triclabendazole content is within specification at 25°C and 30°C after 18 months for batch No 1, and after 9 months for batches No 2 and No 3.

It can be concluded that one batch of Moxidectin and Triclabendazole oral drench formulation maintained a concentration of both moxidectin and triclabendazole that is within specification at storage temperatures of 25°C and 30°C for a period of 18 months. The stability study data presented suggests that will remain stable for at least 18 months when stored at or below 30°C. The remaining two batches have shown chemical stability for 9 months at both 25 and 30°C, with the exception of one batch being below specification at 30°C.

### EXAMPLE VI

### SYRINGABILITY TESTS

The preferred formulation which exhibited the necessary chemical stability was then tested for syringability, *i.e*., for its suitability as an oral drench. Testing concluded that the aforementioned formulation was suitable for use as an oral drench and was competitive in this regard with currently available liver fluke oral drenches on the market.

## Claims

1. An anthelmintic oral drench composition comprising 0.01 5% w/v of a milbemycin, 1-10% w/v triclabendazole and a non-surfactant rheology modifier.

2. A composition according to Claim 1 wherein the rheology modifier is an inorganic rheology modifier.

3. A composition according to Claim 1 wherein the rheology modifier is Laponite®.

4. A composition according to Claim 1 wherein the rheology modifier is Laponite RDS®.

5. A composition according to any one of Claims 1 to 4 comprising 0.1 - 2% w/v of rheology modifier.

6. A composition according to any one of Claims 1 to 5 wherein the predetermined quantity of rheology modifier is sufficient to render the viscosity no greater than 2000 centipoises.

7. A composition according to Claim 6 wherein the predetermined quantity of rheology modifier is sufficient to render the viscosity no greater than 1500 centipoises.

8. A composition according to Claim 7 wherein the predetermined quantity of rheology modifier is sufficient to render the viscosity no greater than 1000 centipoises.

9. A composition according to Claim 8 wherein the predetermined quantity of rheology modifier is sufficient to render the viscosity no greater than 500 centipoises.

10. A composition according to any one of Claims 1 to 9 further comprising up to 0.1% selenium.

11. A veterinary oral drench composition comprising:
0.01-5% w/v a milbemycin;
sufficient amounts of one or more organic solvents and one or more surfactants to enable the dissolution of the milbemycin;
1-10% w/v triclabendazole;
0.1-2% w/v of a non-surfactant rheology modifier,
40-80% water; and optionally, solvents, surfactants, buffering agents, and preservatives.

12. A method of rendering an anthelmintic composition comprising a milbemycin and triclabendazole suitable for use as an oral drench, comprising adding a predetermined quantity of non-surfactant rheology modifier, wherein the rheology modifier is Laponite RDS®.

13. A method according to claim 12 wherein the predetermined quantity of rheology modifier is sufficient to render the viscosity in the range of 1650 - 2050 MPas.

14. A method according to claim 12 or claim 13 wherein said anthelmintic composition includes up to 0.1% selenium.

15. A method according to any one of claims 12. to 14 comprising adding sufficient quantities of one or more organic solvents or one or more surfactants to enable dissolution of the milbemycin.

16. A method of producing a veterinary oral drench composition comprising combining:
0.01-5% w/v of a milbemycin;
sufficient amounts of one or more organic solvents and one or more surfactants to enable the dissolution of the milbemycin;
1-10% w/v triclabendazole;
0.1-2% w/v of a non-surfacant rheology modifier;
40-80% water; and optionally, solvents, surfactants, buffering agents, and preservatives.

## Patentansprüche

1. Anthelmintische orale Drench-Zusammensetzung umfassend zu 0,01-5 Gew./Vol.-% ein Milbemycin, zu 1-10 Gew./Vol.-% Triclabendazol und einen nicht-obertlächenaktiven Rheologiemodifizierer.

2. Zusammensetzung nach Anspruch 1, wobei der Rheologiemodifizierer ein anorganischer Rheologiemodifizierer ist.

3. Zusammensetzung nach Anspruch 1, wobei der Rheologiemodifizierer Laponit^{®} ist.

4. Zusammensetzung nach Anspruch 1, wobei der Rheologiemodifizierer Laponit RDS^{®} ist.

5. Zusammensetzung nach irgendeinem der Ansprüche 1 bis 4, umfassend zu 0,1-2 Gew./Vol.-% einen Rheologiemodifizierer.

6. Zusammensetzung nach irgendeinem der Ansprüche 1 bis 5, wobei die vorbestimmte Menge des Rheologiemodifizierers ausreicht, um die Viskosität nicht größer als 2000 centipoises zu machen.

7. Zusammensetzung nach Anspruch 6, wobei die vorbestimmte Menge des Rheologiemodifizierers ausreicht, um die Viskosität nicht größer als 1500 centipoises zu machen.

8. Zusammensetzung nach Anspruch 7, wobei die vorbestimmte Menge des Rheologiemodifizierers ausreicht, um die Viskosität nicht größer als 1000 centipoises zu machen.

9. Zusammensetzung nach Anspruch 8, wobei die vorbestimmte Menge des Rheologiemodifizierers ausreicht, um die Viskosität nicht größer als 500 centipoises zu machen.

10. Zusammensetzung nach irgendeinem der Ansprüche 1 bis 9, ferner umfassend bis zu 0,1% Selen.

11. Veterinärmedizinische orale Drench-Zusammensetzung umfassend:
zu 0,01-5 Gew./Vol.-% ein Milbemycin;
ausreichende Mengen eines oder mehrerer organischer Lösungsmittel und eines oder mehrerer oberflächenaktiver Stoffe, um die Auflösung des Milbemycins zu ermöglichen;
zu 1-10 Gew./Vol.-% Triclabendazol;
zu 0,1-2 Gew./Vol.-% einen nicht-oberflächenaktiven Rheologiemodifizierer;
zu 40-80% Wasser; sowie gegebenenfalls Lösungsmittel, oberflächenaktive Stoffe, Puffersubstanzen und Konservierungsstoffe.

12. Verfahren zur Anpassung einer anthelmintischen Zusammensetzung umfassend ein Milbemycin und Triclabendazol an die Verwendung als oraler Drench, umfassend den Zusatz einer vorbestimmten Menge eines nicht-oberflächenaktiven Rheologiemodifizierers, wobei der Rheologiemodifzierer Laponit RDS^{®} ist.

13. Verfahren nach Anspruch 12, wobei die vorbestimmte Menge des Rheologiemodifizierers ausreicht, um die Viskosität im Bereich von 1650-2050 mPa.s zu halten.

14. Verfahren nach Anspruch 12 oder Anspruch 13, wobei die anthelmintische Zusammensetzung bis zu 0,1% Selen umfasst.

15. Verfahren nach irgendeinem der Ansprüche 12 bis 14, umfassend den Zusatz ausreichender Mengen eines oder mehrerer organischer Lösungsmittel oder eines oder mehrerer oberflächenaktiver Stoffe, um die Auflösung des Milbemycins zu ermöglichen.

16. Verfahren zur Herstellung einer veterinärmedizinischen oralen Drench-Zusammensetzung, umfassend das Kombinieren von Folgendem:
zu 0,01-5 Gew./Vol.-% ein Milbemycin;
ausreichende Mengen eines oder mehrerer organischer Lösungsmittel und eines oder mehrerer oberflächenaktiver Stoffe, um die Auflösung des Milbemycins zu ermöglichen;
zu 1-10 0 Gew.Nol.-% Triclabendazol;
zu 0,1-2 Gew./Vol.-% einen nicht-oberflächenaktiven Rheologiemodifizierer;
zu 40-80% Wasser; sowie gegebenenfalls Lösungsmittel, oberflächenaktive Stoffe, Puffersubstanzen und Konservierungsstoffe.

## Revendications

1. Composition orale anthelminthique sous forme de breuvage, comprenant une milbémycine à concurrence de 0,01 à 5 % en poids/volume, du triclabendazole à concurrence de 1 à 10 % en poids/volume et un modificateur de la rhéologie non tensioactif.

2. Composition selon la revendication 1, dans laquelle le modificateur de la rhéologie est un modificateur de la rhéologie de type inorganique:

3. Composition selon la revendication 1, dans laquelle le modificateur de la rhéologie est la Laponite®.

4. Composition selon la revendication 1, dans laquelle le modificateur de la rhéologie est la Laponite RDS®.

5. Composition selon l'une quelconque des revendications 1 à 4, comprenant le modificateur de la rhéologie à concurrence de 0,1 à 2 % en poids/volume.

6. Composition selon l'une quelconque des revendications 1 à 5, dans laquelle la quantité prédéterminée du modificateur de la rhéologie est suffisante pour obtenir une viscosité qui n'est pas supérieure à 2000 centipoises.

7. Composition selon la revendication 6, dans laquelle la quantité prédéterminée du modificateur de la rhéologie est suffisante pour obtenir une viscosité qui n'est pas supérieure à 1500 centipoises.

8. Composition selon la revendication 7, dans laquelle la quantité prédéterminée du modificateur de la rhéologie est suffisante pour obtenir une viscosité qui n'est pas supérieure à 1000 centipoises,

9. Composition selon la revendication 8, dans laquelle la quantité prédéterminée du modificateur de la rhéologie est suffisante pour obtenir une viscosité qui n'est pas supérieure à 500 centipoises.

10. Composition selon l'une quelconque des revendications 1 à 9, comprenant en outre du sélénium jusqu'à concurrence de 0,1 %.

11. Composition vétérinaire orale sous forme de breuvage, comprenant :
une milbémycine à concurrence de 0,01 à 5 % en poids/volume ;
des quantités suffisantes d'un ou de plusieurs solvants organiques et d'un ou de plusieurs agents tensioactifs pour obtenir la dissolution de la milbémycine ;
du triclabendazole à concurrence de 1 à 10 % en poids/volume ;
un modificateur de la rhéologie non tensioactif à concurrence de 0,1 à 2 % en poids/volume
de l'eau à concurrence de 40 à 80 % ; et, de manière facultative, des solvants, des agents tensioactifs, des tampons et des conservateurs.

12. Procédé pour rendre une composition anthelminthique, comprenant une milbémycine et du triclabendazole, appropriée pour une utilisation sous forme d'une breuvage oral, comprenant l'addition d'une quantité prédéterminée d'un modificateur de la rhéologie non tensioactif, dans lequel le modificateur de la rhéologie est la Laponite RDS®.

13. Procédé selon la revendication 12, dans lequel la quantité prédéterminée du modificateur de la rhéologie est suffisante pour obtenir une viscosité dans la plage de 1650 à 2050 mPa.s.

14. Procédé selon la revendication 12 ou 13, dans lequel ladite composition anthelminthique englobe du sélénium jusqu'à concurrence de 0,1 %.

15. Procédé selon l'une quelconque des revendications 12 à 14, comprenant l'addition de quantités suffisantes d'un ou de plusieurs solvants organiques ou d'un ou de plusieurs agents tensioactifs pour obtenir la dissolution de la milbémycine.

16. Procédé de préparation d'une composition vétérinaire orale sous forme de breuvage, comprenant le fait de combiner:
une milbémycine à concurrence de 0,01 à 5 % en poids/volume ;
des quantités suffisantes d'un ou de plusieurs solvants organiques et d'un ou de plusieurs agents tensioactifs pour obtenir la dissolution de la milbémycine ;
du triclabendazole à concurrence de 1 à 10 % en poids/volume ;
un modificateur de la rhéologie non tensioactif à concurrence de 0,1 à 2 % en poids/volume ;
de l'eau à concurrence de 40 à 80 % ; et, de manière facultative, des solvants, des agents tensioactifs, des tampons et des conservateurs.
